# EUROPEAN PATENT APPLICATION

(11) **EP 4 552 664 A1**
(43) Date of publication of application: **14.05.2025**
(21) Application number: 23208606.6
(22) Date of filing: 08.11.2023
(51) Int. Cl.: A61M 1/00

(54) **PROCEDURE AND DEVICE FOR THE TREATMENT OF OSTEOMYELITIS IN LONG BONES BY NEGATIVE PRESSURE**

(71) Applicant: Schenone, Griselda Liliana, B1678 CVD Caseros, Buenos Aires (AR)
(72) Inventor: Schenone, Griselda Liliana, B1678 CVD Caseros, Buenos Aires (AR)
(74) Representative: Durán-Corretjer, S.L.P.

(57) **Abstract**

A procedure for the treatment of osteomyelitis in long bones comprising the steps of:
- approaching the bone canal of a long bone either at its distal or proximal end through a wound made on a patient;
- reaming said bone canal to a maximum diameter of approximately 12 mm (0.47 in) without affecting the cortical layer;
- taking samples for culture;
- cleaning and aspirating the contents of said bone canal;
- inserting into said bone canal, a multi-perforated plastic cannula (1) with holes (2) therein by means of a metal guide (3) arranged inside and having the exterior thereof covered with a sponge (4);
- removing said metal guide (3);
- arranging an additional portion of sponge in said wound in such a way that one portion is placed deep in contact with said a multi-perforated plastic cannula (1) coated with a sponge (4), and another portion emerges from said wound;
- partially closing said wound with sutures;
- fixing a self-adherent absorption disc that includes an aspiration hose upon said additional portion of sponge emerging from said wound;
- coating said wound with a self-adherent film to form a complete seal;
- connecting by means of a connector said aspiration hose to a collection hose which is connected to an external graduated container;
- connecting said external graduated container to a suction pump;
- turning on said suction pump;
- absorbing and transferring the fluid generated within said bone canal continuously to said external graduated container;
- measuring the aspirated fluid;
- carrying out the above steps three times every 48 hours; and
- after the third time, completely removing said a multi-perforated plastic cannula covered with said sponge from said wound by re-reaming, washing, and taking cultures for laboratory testing.
This invention also discloses a device for the treatment of osteomyelitis in long bones used in the procedure.

## Description

The present invention refers to a procedure and device for the treatment of osteomyelitis in long bones by negative pressure.

### BACKGROUND OF THE INVENTION

A wide variety of devices for the treatment of osteomyelitis by negative pressure are currently available.

CN113456924A discloses a method of treating osteomyelitis and a lavage drainage device thereof. The lavage drainage device comprises an inner drainage cannula and an outer lavage cannula disposed outside the inner drainage cannula as a sheath. A closed lavage cavity is formed between the inner drainage cannula and the outer lavage cannula, and a lavage opening communicates with the lavage cannula. A cavity and a liquid inlet are formed in the external lavage cannula wall. The front end of the inner drainage cannula extends out of the outer lavage cannula and is provided with a backflow inlet; the liquid inlet is used to introduce lavage liquid into the lavage cavity, the rear end of the internal drainage cannula is connected with a negative pressure equipment, and the lavage liquid sprayed out of the lavage opening sequentially passes through the reflux opening and the internal drainage cannula under the negative pressure effect provided by the negative pressure equipment and is discharged from the rear end of the internal drainage cannula. According to the lavage drainage device, the lavage structure and drainage structure are combined with each other so as to overcome the defects of large trauma to an operation area, non-smooth drainage and the like caused by the separate effect of traditional lavage and drainage. A new lavage treatment during and after clinical osteomyelitis surgery is also provided.

This device does not differ from conventional treatment for the treatment of osteomyelitis where lavage and suction are performed separately. It involves the union of two cannulas, external and internal, with different diameters. This device comprising unified cannulas does not ensure the removal of all contaminated tissues since the internal cannula is designed to aspirate the fluid, and it does so through the more distal end with a single suction port that can be easily occluded.

CN206482909U describes a negative pressure lavage and draining device for osteomyelitis, which includes a drainage cannula and a lavage cannula and a T-shaped medical sponge, the wear of the lavage cannula affects the inner cavity of the drainage cannula, leaving the circulation-free space between the outer wall of the lavage cannula and the inner face of the drainage cannula. The outer wall of the upstream drainage cannula is provided with the inlet cannula which is used to connect one end of the drainage cannula, the end of the inlet cannula being fixed on the outer wall of the drainage cannula. The lavage cannula penetrates the necrotic tissue and at the same time, the necrotic tissue comes out easily through the drainage cannula.

The purpose of this device is to wash the contaminated area with a solution, assisted by a valve that allows the liquid to enter, and it is closed when a sensor is activated. This liquid remains in the distal zone of the lavage cannula and on the T-shaped sponge, causing a local effect. After a pre-determined time, another valve allows the starting of the suction system to extract the fluid that has entered by the action of the negative pressure, taking it to the outside. The T-shaped sponge turns out to be a common zone for both the incoming and outgoing fluids.

With this device, the lavage performed is not actually effective for infection control. The design thereof turns out to be complex to use since each of the steps must be set at different times, preset by the treating surgeon. The sensors prove to be ineffective because they allow the overflow of the incoming fluids. There is also the possibility of fluid leakage due to insufficient control of the valves, which generates reddening, maceration, and contamination of the surrounding healthy skin, further aggravating the pre-existing infection. This fluid leakage causes the negative pressure system to rupture and increases wound sequestration. The cannula system, combined with fluid inflow and outflow, proves to be complex for the surgeon to use and turns out to be inefficient for the results expected to be achieved with the design thereof.

The device of the invention is a combination of a multi-perforated cannula with opposed alternating holes, said cannula being covered all along by a sponge, and the total diameter of the device is approximately 10 mm (0.39 in). The positioning of the device inside the bone is done with the help of a metal guide which is then removed. The cannula holes are placed approximately 20 mm (0.78 in) apart in an alternating and opposite arrangement, as mentioned above. This arrangement, together with the sponge, allows the surface area to be enlarged to transport all the fluids generated inside the bone more efficiently. The sponge lattice, by the action of the negative pressure, retains the larger particles preventing the infection from being perpetuated by sequestration within the bone canal. In addition, the device of the invention has been developed to be used with negative pressure without using irrigation to clean the canal while the surgical procedure of excision and lavage is previous to the placement of the device of the invention.

Thus, the object of the present invention is a procedure for the treatment of osteomyelitis in long bones **characterized in that** it comprises the following steps:
- approaching the bone canal of a long bone either at its distal or proximal end through a wound made on a patient;
- reaming said bone canal to a maximum diameter of approximately 12 mm (0.47 in) without affecting the cortical layer;
- taking samples for culture;
- cleaning and aspirating the contents of said bone canal;
- inserting into said bone canal, a multi-perforated plastic cannula with holes therein by means of a metal guide arranged inside and having the exterior thereof covered with a sponge;
- removing said metal guide;
- arranging an additional portion of sponge in said wound in such a way that one portion is placed deep in contact with said a multi-perforated plastic cannula coated with a sponge, and another portion emerges from said wound;
- partially closing said wound with sutures;
- fixing a self-adherent absorption disc that includes an aspiration hose upon said additional portion of sponge emerging from said wound;
- coating said wound with a self-adherent film to form a complete seal;
- connecting, by means of a connector, said aspiration hose to a collection hose which is connected to an external graduated container;
- connecting said external graduated container to a suction pump;
- turning on said suction pump;
- absorbing and transferring the fluid generated within said bone canal continuously to said external graduated container;
- measuring the aspirated fluid;
- carrying out the above steps three times every 48 hours; and
- after the third time, completely removing said a multi-perforated plastic cannula covered with said sponge from said wound by re-reaming, washing, and taking cultures for laboratory testing.

Still another object of the invention is a device for the treatment of osteomyelitis in long bones by negative pressure used in the above procedure, **characterized in that** it comprises:
- a multi-perforated plastic cannula;
- a metal guide placed inside said a multi-perforated plastic cannula; and
- a sponge covering said a multi-perforated plastic cannula.

### BRIEF DESCRIPTION OF THE FIGURE

Figure 1 illustrates a schematic view of the device of the invention.
Figure 2 illustrates a multi-perforated plastic cannula with holes and metal guide inside the cannula.
Figure 3 illustrates a multi-perforated plastic cannula with sponge covering.
Figure 4 illustrates an insertion, wound Closure, disc and external connector.
Figure 5 illustrates (A) canal suction and (B) bacterial inoculation.

### DETAILED DESCRIPTION OF THE INVENTION

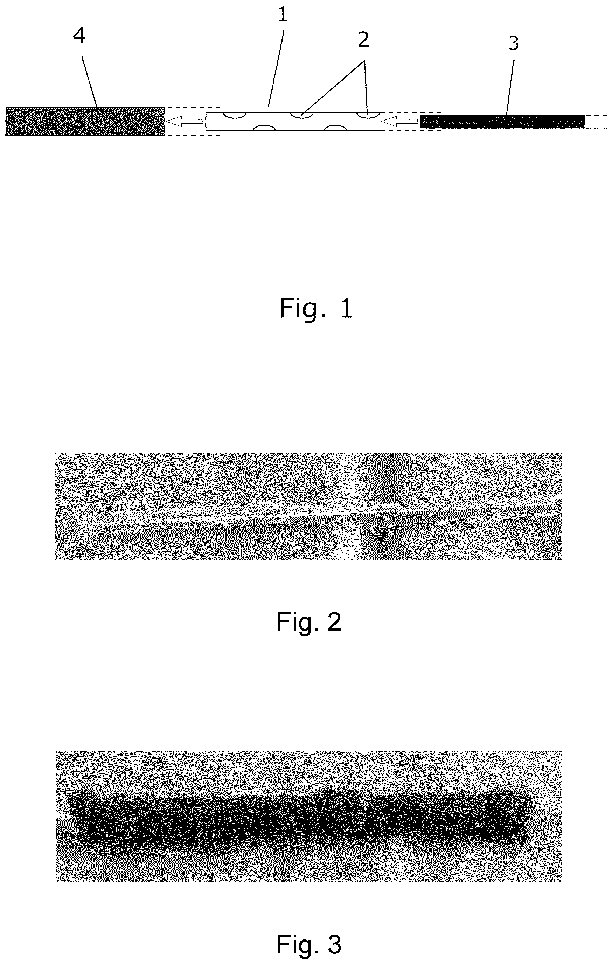

Figure 1 illustrates the device of the invention which comprises a multi-perforated plastic cannula 1 with holes 2 which is introduced into the distal or proximal end of the bone canal of long bones with osteomyelitis to aspirate and remove, by applying negative pressure, fluids, and particles (biological substances) to perform a more effective surgical toilet. The holes 2 of said a multi-perforated plastic cannula 1 facilitate the entry of the fluids generated inside the bone canal and the transport of the same by means of a negative suction pump to an external container. Said holes 2 are alternating and oppositely arranged approximately every 20 mm (0.78 in).

Likewise, said a multi-perforated plastic cannula 1 is covered by a sponge 4, preferably made from 600 microns hydrophobic non-collapsible cross-linked polyurethane, which allows enhancement of the suction surface of all the fluids generated inside the infected long bones.

Said a multi-perforated plastic cannula 1 covered with said sponge 4 has a metal guide 3 arranged inside to facilitate the insertion thereof in said bone canal.

Initially, the bone canal of a long bone is approached through a wound made on the patient, either at its distal or proximal end by means of a wound made on a patient. Subsequently, the bone canal is reamed to a maximum diameter of approximately 12 mm (0.47 in) without affecting the cortical layer, samples are taken for culture, and finally the contents of the bone canal are washed and aspirated.

Subsequently, said a multi-perforated plastic cannula 1 covered with said sponge 4 is inserted in said bone canal aided with said metal guide 3, the total diameter of the device being approximately 10 mm (0.39 in), i.e., approximately 2 mm (0.07 in) less than the diameter of said bone canal after the above-mentioned reaming. This difference in 2 mm (0.07 in) ensures easy placement and removal, preventing the adherence of the material to the internal wall of the bone.

Once said a multi-perforated plastic cannula 1 covered with said sponge 4 is inserted, said metal guide 3 is removed, proceeding to the partial closure of the wound made on the patient with sutures, prior arrangement of an additional portion of sponge (not illustrated) in said wound so that a portion is inserted deeply in contact with said cannula covered with sponge, and another portion emerges from said wound. In this way, a small opening is left through which said additional portion of the sponge emerges, and a self-adherent absorption disc (not illustrated) rests thereon. An absorption hose (not illustrated) which conforms a single unit with said self-adherent absorption disc is connected, by means of a connector, to a collection hose (not illustrated) which, in turn, is connected to an external graduated container (not illustrated).

Said external graduated container is connected to a suction pump, wherein the suction or negative pressure is kept constant at approximately 125 mm Hg or 167 HPa.

Subsequently, said suction pump is turned on in order to continuously aspirate and transfer the fluid generated inside said bone canal to said external graduated container, and thus, the aspirated fluid may be measured.

All these steps are repeated three times every 48 hours and, after the third time, said a multi-perforated plastic cannula 1 covered with said sponge 4 is completely removed from said wound by performing a new reaming and taking cultures for laboratory testing.

Now, the Applicant wishes to illustrate the invention by means of some tests carried out on sheep.

### TESTS CARRIED OUT ON SHEEP USING THE DEVICE OF THE INVENTION

In order to check the positive outcome of the treatment for chronic osteomyelitis, experimental development in sheep was considered [1 ;2;3]. Such sheep were traceable animals in terms of sanitary control, vaccination, deworming. The Hospital's Research Committee, following the regulations of the Animal Protection Association, authorized the use of 8 (eight) sheep for this two-step study [4;5]. Three of them were used as controls, without infection. The complete scheme (including germ inoculation) was performed on the remaining five sheep. During the anesthetic induction of the first step, a dose of prophylactic ATB was administered 1 hour before surgery, to avoid the fatal sepsis of the animal. Next, *St. aureus* (29213 0.5 Mc Facland) was inoculated through a perforation in the femoral cortex. Once this was done, the sheep completed a waiting period of 30 days for the replication of the bacteria. Once this period was over, the second step was carried out. It consisted of the toilet, excision, and culture sampling, adding the placement of the device of the invention, implemented every 48 hours. The results obtained by laboratory and pathological anatomy testing were recorded.

**Object:** To find both local and distance effects produced by the use of the device of the invention.

**Material and Method:** In this test, 8 (eight) sheep were used, all of them traceable. The testing was carried out in two steps. First step: Involvement of the femur bone. Second step: Reaming treatment, toilet, culture sampling, and placement of the device of the invention.

**Outcome:** The procedure was well tolerated every 48 hours. No blood transfusion was necessary due to the wound discharge caused by the negative pressure inside the canal. Infection values were shown to decrease in the controls performed in combination with the use of antibiotics.

### Introduction

Based on the outcome initially obtained in a patient with chronic osteomyelitis as a result of a compound fracture with infection of over three years and given the repeated failure of antibiotic therapy and traditional toilet, it was decided to use the device of the invention, adapting it to the anatomy of the bone and insert it in the bone canal. Due to the satisfactory result, the same system was applied to other patients. Given the lack of background in the medical literature, an experimental study was carried out on animals to verify and confirm the validity of this procedure for the treatment of recurrent infections. Resources and techniques similar to those used in humans were used.

The literature describes a wide variety of medium-sized animals to study the development of infections. The sheep was selected due to its ease of handling, low-maintenance requirements, and docile nature. The size of its femur bone allows the use of the same surgical instruments applicable to humans without major changes that adjust to the anatomy of the animal. According to medical protocols, 6 to 12 weeks is established as an average period for the development of infections. In our case, due to unexpected results at the beginning, changes had to be made because of the rapid deterioration produced by the long wait which caused the subsequent septicemia and death of the animal.

The objective of this investigation is to verify the effectiveness of the application of the device of the invention for the treatment of recurrent infections in diaphyseal fractures, complications and collateral effects thereof (adherence of the material to the bone, fat embolism, difficulties in the placement and removal of the placed material). It is also to verify the reduction of the titer of microorganisms in the samples obtained from the animals.

### Materials and Methods

### Components of the Present Device

A multi-perforated plastic cannula with Holes: The cannula perforations enable the easy entry of the fluids generated inside the bone canal and the transport of the same by negative suction into an external graduated container to control the discharge obtained and to measure the aspirated volume, as shown in Figure 2.

Sponge Covering: The plastic cannula has a 600-micron hydrophobic, non-collapsible, cross-linked polyurethane covering that boosts the suction surface which absorbs all the fluids generated inside long bones, as shown in Figure 3.

Metal Guide: It is inserted along the multi-perforated cannula which has been already coated with the sponge, for the easy placement of the device of the invention inside the bone canal. Immediately after the device is placed inside the bone canal, the metal guide is removed.

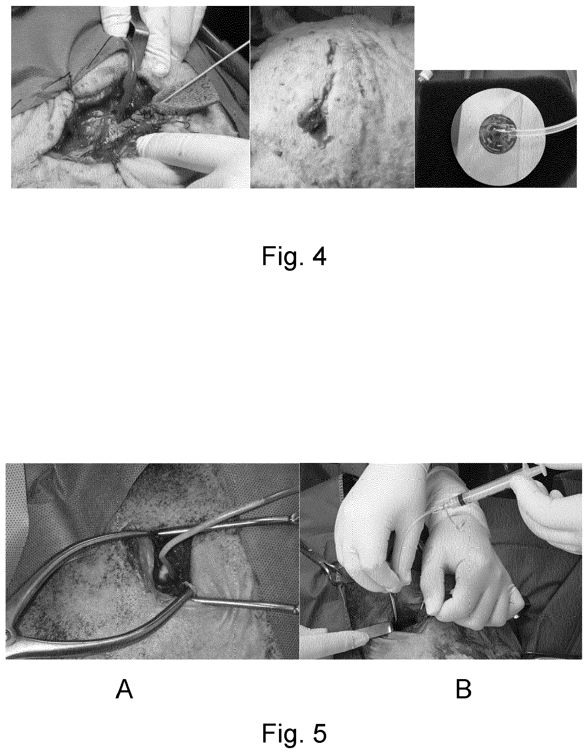

An additional portion of the sponge is placed in the wound in such a way that a portion remains deep in contact with said sponge-coated cannula, and another portion emerges from said wound, to subsequently perform a partial closure of said wound by means of sutures. Externally to said wound and on said additional portion of sponge protruding therefrom, a self-adherent absorption disk is provided which includes an aspiration hose. This latter is further connected, by means of a connector, to a collector hose which, in turn, is connected/joined to an external graduated container, which allows measuring the wound discharge achieved by the negative pressure suction generated by a suction pump to which said external graduated container is connected. The negative pressure is kept constant at approximately 125 mm Hg or 167 HPa, preventing leakage at the connectors. The above is shown in Figure 4.

Eight adult sheep between the ages of 3 and 5, with an average weight of 55 kg (121 lb), were used in this test. The sheep were obtained from a farm, 48 hours before the surgery for the adaptation thereof to the environment before the surgical procedure. The sheep were fasted for 24 hours before surgery. Inside the pen, anesthetic induction was performed with shaving of the area to be treated before transferring the sheep to the procedure room. In the same place, 1 g (0.03 oz) of ATB Cefazolin was administered. The purpose of this pre-surgery administration was to establish a localized infection in the limb, preventing septicemia. On the operating table, the proximal femur was visualized having previously placed sterile fields and with the help of the image intensifier. The lateral aspect of the femur was reamed through the cortex using a 3.5 mm (0.13 in) drill bit. The content of the bone marrow was aspirated with a catheter and a syringe to increase the space and the subsequent inoculation of bacteria into the bone, as shown in Figure 5. After wound closure, the sheep was taken to a pen where it recovered from anesthesia until completing a 30-day guarding period for bacterial development, under the control of the veterinary staff in charge.

This experiment sought to develop a degree of deep infection achieved through direct inoculation of bacteria into the bone.

After this period (30 days), the sheep was taken again to the operating room for the completion of the second step. The bone canal was approached through the pit. An 8 mm (0.31 in) long reaming was performed until a maximum diameter of approximately 12 mm (0.47 in) was reached, without affecting the cortical layer. Samples were taken for culture, following a canal lavage and placement of the device of the invention whose diameter was approximately 10 mm (0.39 in). This difference in 2 mm (0.07 in) ensured the easy placement and removal thereof, preventing the adherence of the material to the internal bone wall. The suction pump with negative pressure was kept operative at approximately 125 mm Hg or 167 HPa. The aspirated material volume was continuously transferred to an external graduated container where the wound discharge could be measured. These steps were performed three times every 48 hours. On the third time in the operating room, the device was completely removed through a new reaming, and cultures were taken for laboratory testing. Post-mortem samples were taken from the different organs for pathological anatomy testing.

### Outcome:

The designed study started with three sheep, which were inoculated in the first step with *St. aureus* through the lateral aspect of the femur. They were then taken to a large pen and kept for observation for 1 month. During the first 2 weeks, the sheep behaved normally. In the third week, they exhibited changes in posture and gait as well as in their general mood with loss of body mass, becoming progressively more lethargic with a decrease in their normal motility. The rapid deterioration of their general condition forced us to cull one of the three sheep without treatment. The other two received the treatment through the device of the invention according to the designed protocol. In the operating room, it was found that the sheep had developed a deep infection and had organized tissue showing evidence of the initial host response. In the 3 specimens, a fracture was found at the inoculation point of the germ. Only 2 of them received treatment with the device of the invention. The third sheep was euthanized because of its poor general condition. This finding was the answer to the first question: Why did the hind legs of the three sheep start showing weaker support at the beginning of the third week?

The pathological anatomy of the fracture site showed hemorrhage, focal necrosis, and acute inflammation.

Based on this result, the initial protocol was modified in three basic points:
1) In contrast to the initial project, only one control sheep was used due to the limited number of authorized specimens.
2) The inoculation point of the germ was changed from the lateral aspect to the bone pit at the femoral neck base, in a longitudinal and descending direction of the bone to avoid possible fractures.
3) The guarding period for the development of the disease was reduced from one month to one week before the application of the therapy by means of the device of the invention. This change in time was introduced to compensate for the rapid deterioration of the sheep that had begun in the third week. Apart from these modifications, the initial protocol was kept as it was.

This first experiment performed on sheep every 48 hours with the device of the invention was well tolerated in all cases, not significantly affecting the general clinical condition of the animals.

No blood transfusions were needed for the recovery of the animals.

The hematocrit and hemoglobin values obtained in the laboratory tests dropped between 30% and 50% in the first surgery. In the second and third surgeries, the value dropping was maintained at approximately a 10% rate. We believe that the removal of the medullary tissue during the first reaming caused this difference, while in the other two surgeries, the reaming only had the effect of removing the contents of the bone canal (i.e., fluids and possible rests of the device of the invention adhered to the bone walls). This could explain the decrease in the dropping of values between surgeries (see Table 1).

White blood cell count decreased in all cases. Although some modifications were observed, it remained below the initial value.

The degree of infection was determined by the colony count of the reaming material obtained during surgery, showing variable decreasing changes (see Table 1). In one case where no antibiotic (ATB) was administered, the values showed a moderate increase.

The pathological anatomy examinations showed the following findings: bone and soft tissue (necrosis-hemorrhage inflammatory tissue (3/8)), lung (bronchopneumonia and bronchial secretion (2/8)), liver (lymphocytic infiltrate (1/8)), skeletal muscle tissue (parasites (2/8)), spleen (parenchymal congestion (1/8)), heart and kidney were normal. Fat embolism was evaluated as a possible complication from reaming, and was not found in any of the cases tested. This shows that repeated reaming every 48 hours does not increase the development of fat embolism.

There was no damage observed due to the device of the invention adhering to the internal walls of the femur. The volume of the discharge by suction of the bone canal did not exceed 300 ml.

### Discussion:

The development of the device of the invention, and the application thereof in sheep had the purpose of validating the efficacy of such development at an intramedullary level. It was created as a response to the search for infection control by relapses where historical treatments had been proven unsuccessful.

The results in sheep were analogous to those obtained in patients. This leads us to affirm that the use of the device of the invention could enhance, by negative suction, the removal of the sequestering elements inside the bone canal that would hinder the proper efficacy of antibiotics. By reducing bacteria within the bone canal, there is a lower likelihood of the formation of biofilm commonly associated with infected areas.

The findings shown in Table 1 confirm the decreasing titration of microorganisms with the application of the device of the invention. In the same way that the diagnoses are confirmed by the varied sampling according to Parvizi's guidelines (between four and five, minimum in humans), the same scheme was used in sheep to obtain an equivalence in the results of this experiment [22].

Although it is not an absolute procedure, the device of the invention proved to be valid as an additional treatment for the management of infections in long bones.

In the sheep organs observed by pathological anatomy, no evidence of fat embolism was found by frequent reaming. In contrast to the associated risk of fat embolism in humans already described in the medical literature, (sic).

It was not possible to find any traces of the VAC device attached to the walls of the bone.

The sheep that did not receive antibiotics (ATB) had increased white blood cell counts and colony growth per field (see Table 1). This last observation confirms that the device of the invention alone is insufficient for infection control.

### Conclusion:

This experiment in sheep would allow us to conclude that the application of the device of the invention, together with the excision and toilet of the area affected by osteomyelitis, would enhance the antibiotic action. This new approach will help to prevent unnecessary expenses from repeated treatments and prolonged hospital stays.

**Table 1**

| Sheep 1 | | | Sheep 2 | | | Sheep 3 | | | Sheep 4 (control) | | |
|---|---|---|---|---|---|---|---|---|---|---|---|
| 5.8 x 10⁷ | | | 6.0 x 10² | | | Euthanized | | | | | |
| 2.4 x 10⁷ | | | NA | | | | | | | | |
| 2.0 x 10⁷ | | | NA | | | | | | | | |
| HCT | HGB | WBC | HCT | HGB | WBC | HCT | HGB | WBC | HCT | HGB | WBC |
| 39 | 11 | 11700 | 40 | 11.3 | 9700 | - | - | - | 40 | 11.7 | 20700 |
| 20 | 5.5 | 5500 | 29 | 8.1 | 6600 | - | - | - | 30 | 8.9 | 15300 |
| 19 | 5.5 | 6000 | 27 | 7.5 | 6000 | - | - | - | 28 | 8.0 | 15000 |
| | | | | | | | | | | | |

| Sheep 5 (without antibiotics) | | | Sheep 6 | | | Sheep 7 | | | Sheep 8 | | |
|---|---|---|---|---|---|---|---|---|---|---|---|
| 4.5 x 10² | | | 1.4 x 10⁴ | | | 1.2 x 10° | | | NA | | |
| 2.0 x 10⁴ | | | NA | | | 2.1 X 10³ | | | NA | | |
| 1.2 x 10⁵ | | | NA | | | 6.4 X 10⁴ | | | NA | | |
| HCT | HGB | WBC | HCT | HGB | WBC | HCT | HGB | WBC | HCT | HGB | WBC |
| 20 | 5.4 | 2700 | 30 | 8.9 | 15300 | 29 | 8.5 | 11000 | 15 | 4.1 | 3500 |
| 17 | 4.7 | 3200 | 31 | 9.3 | 11700 | 22 | 6.1 | 6000 | 14 | 4 | 2500 |
| 16 | 4.5 | 4400 | 30 | 8.7 | 9000 | 20 | 5.7 | 5800 | 16 | 4.5 | 2500 |

| | | | | | | | | | | | |
|---|---|---|---|---|---|---|---|---|---|---|---|
| HCT: Hematocrit HGB: Hemoglobin WBC: White Blood Cells (indicative of an infection present) | | | | | | | | | | | |

In the trial, it is essential to know how much the HCT and HGB values drop during each surgery to determine the necessity of a blood transfusion. The data indicates a 40% decrease in the first surgery and 10% in the subsequent surgery. The first surgery's drop value is more significant because, in the case of sheep with the reaming of the bone canal, most of the cancellous bone was removed. In the present trial, the sheep did not receive a transfusion to restore the declining hematopoietic function. However, in the case of human patients, these values must be restored by transfusions during hospitalization. White blood cell counts determine the degree of infection. The reading of the results shows that they decrease in all cases. Even when some variant appears, they are below the initial value. This shows the positive aspect of using negative pressure with the device of the invention, combined with reaming and lavage of the bone canal. Note that sheep 5 did not receive antibiotics as its WBC (white blood cells) count increased.

## Claims

1. A procedure for the treatment of osteomyelitis in long bones **characterized in that** it comprises the steps of:
- approaching the bone canal of a long bone either at its distal or proximal end through a wound made on a patient;
- reaming said bone canal to a maximum diameter of approximately 12 mm (0.47 in) without affecting the cortical layer;
- taking samples for culture;
- cleaning and aspirating the contents of said bone canal;
- inserting into said bone canal, a multi-perforated plastic cannula (1) with holes (2) therein by means of a metal guide (3) arranged inside and having the exterior thereof covered with a sponge (4);
- removing said metal guide (3);
- arranging an additional portion of sponge in said wound in such a way that one portion is placed deep in contact with said a multi-perforated plastic cannula (1) coated with a sponge (4), and another portion emerges from said wound;
- partially closing said wound with sutures;
- fixing a self-adherent absorption disc that includes an aspiration hose upon said additional portion of sponge emerging from said wound;
- coating said wound with a self-adherent film to form a complete seal;
- connecting by means of a connector said aspiration hose to a collection hose which is connected to an external graduated container;
- connecting said external graduated container to a suction pump;
- turning on said suction pump;
- absorbing and transferring the fluid generated within said bone canal continuously to said external graduated container;
- measuring the aspirated fluid;
- carrying out the above steps three times every 48 hours; and
- after the third time, completely removing said a multi-perforated plastic cannula covered with said sponge from said wound by re-reaming, washing, and taking cultures for laboratory testing.

2. The procedure of claim 1, **characterized in that** said suction pump is maintained at a constant pressure of about 167 HPa (125 mm HG).

3. A device for the treatment of osteomyelitis in long bones by negative pressure used in the procedure of claim 1, **characterized in that** it comprises:
- a multi-perforated plastic cannula (1) with holes (2);
- a metal guide (3) placed inside said a multi-perforated plastic cannula (1); and
- a sponge (4) covering said a multi-perforated plastic cannula (1).

4. The device of claim 3, **characterized in that** said sponge (4) is made from 600-micron hydrophobic non-collapsible cross-linked polyurethane.

5. The device of claim 3, **characterized in that** said holes (2) are placed approximately 20 mm (0.78 in) apart in an alternating and opposite arrangement.

6. The device of claim 1, **characterized in that** the diameter thereof is approximately 10 mm (0.39 in).
